# EUROPEAN PATENT SPECIFICATION

(11) **EP 2 984 101 B1**
(45) Date of publication and mention of the grant of the patent: **19.08.2020**
(21) Application number: 14778974.7
(22) Date of filing: 04.04.2014
(51) Int. Cl.: C07K 1/14, C12Q 1/68, C12N 15/10

(54) **KITS AND METHODS FOR ISOLATING PROTEIN FROM BIOLOGICAL AND ENVIRONMENTAL SAMPLES**
KITS UND VERFAHREN ZUR ISOLIERUNG VON PROTEIN AUS BIOLOGISCHEN UND UMWELTPROBEN
KITS ET MÉTHODES DESTINÉS À ISOLER UNE PROTÉINE D'ÉCHANTILLONS BIOLOGIQUES ET ENVIRONNEMENTAUX

(30) Priority: 05.04.2013 US 201361809237 P
(43) Date of publication of application: 17.02.2016
(73) Proprietor: Qiagen Sciences, LLC, Germantown, MD 20874 (US)
(72) Inventor: CALLAHAN, Heather, San Diego, CA 92127 (US); KENNEDY, Suzanne, Houston, TX 77006-4020 (US); BROLASKI, Mark, Encinitas, CA 92024 (US)
(74) Representative: Roth, Carla
(86) International application number: PCT/US2014/032995
(87) International publication number: WO 2014/165769

(56) References cited:
- EP-A1- 2 199 295
- WO-A1-2009/140313
- WO-A2-2006/130720
- WO-A2-2009/025690
- WO-A2-2012/135081
- US-A- 4 748 234
- US-A- 5 081 010
- US-A- 5 665 359
- US-A- 5 834 282
- US-A1- 2003 153 083
- US-A1- 2009 111 159
- US-B1- 7 429 648
- Domenick G. Grasso ET AL: "Overexpression and Purification of Mammalian Mitochondrial Translational Initiation Factor 2 and Initiation Factor 3" In: "METHODS IN ENZYMOLOGY", 1 January 2007 (2007-01-01), ACADEMIC PRESS, US, XP055328434, ISSN: 0076-6879 vol. 430, pages 59-78, DOI: 10.1016/S0076-6879(07)30004-9, * the whole document, in particular "buffer 3A" in section 2.4 and "Luria Broth" in section 2.1 *
- YEATES C ET AL: "Methods for microbial DNA extraction from soil for PCR amplification", BIOLOGICAL PROCEDURES ONLINE, BIOLOGICAL PROCEDURES ONLINE, WATERLOO, CA, vol. 1, no. 1, 14 May 1998 (1998-05-14), pages 40-47, XP002990749, ISSN: 1480-9222, DOI: 10.1251/BPO6
- SCHNEEGURT M ET AL: "DIRECT EXTRACTION OF DNA FROM SOILS FOR STUDIES IN MICROBIAL ECOLOGY", CURRENT ISSUES IN MOLECULAR BIOLOGY, CAISTER ACADEMIC, WYMONDHAM, GB, vol. 5, no. 1, 1 January 2003 (2003-01-01) , pages 1-08, XP008044065, ISSN: 1467-3037
- DANA WILLNER ET AL: "Comparison of DNA Extraction Methods for Microbial Community Profiling with an Application to Pediatric Bronchoalveolar Lavage Samples", PLOS ONE, vol. 7, no. 4, 13 April 2012 (2012-04-13), page e34605, XP055329239, DOI: 10.1371/journal.pone.0034605
- KARUNA CHOUREY ET AL: "Direct Cellular Lysis/Protein Extraction Protocol for Soil Metaproteomics", JOURNAL OF PROTEOME RESEARCH., vol. 9, no. 12, 3 December 2010 (2010-12-03), pages 6615-6622, XP055329422, US ISSN: 1535-3893, DOI: 10.1021/pr100787q
- Anonymous: "Thermo Scientific Pierce Cell Lysis Technical Handbook", 1 January 2008 (2008-01-01), THERMO SCIENTIFIC PIERCE, XP008180655, * the whole document *
- LUKASZ P. KOZLOWSKI: "Proteome- pI : proteome isoelectric point database", NUCLEIC ACIDS RESEARCH, vol. 45, no. D1, 4 January 2017 (2017-01-04), pages D1112-D1116, XP055422709, ISSN: 0305-1048, DOI: 10.1093/nar/gkw978
- K C Duong-Ly ET AL: "Salting out of proteins using ammonium sulfate precipitation", Methods Enzymol., vol. 541, 1 January 2014 (2014-01-01), pages 85-87, XP055635437,
- MST KAMRUN NAHAR ET AL: "Effect of pH and Salt Concentration on Protein Solubility of Slaughtered and Non-Slaughtered Broiler Chicken Meat", SAINS MALAYSIANA : JOURNAL OF NATURAL SCIENCES, vol. 46, no. 5, 31 May 2017 (2017-05-31), pages 719-724, XP055635438, ISSN: 0126-6039, DOI: 10.17576/jsm-2017-4605-06
- ZAHUCZKY G ET AL: "Cloning of the bovine leukemia virus proteinase in Escherichia coli and comparison of its specificity to that of human T-cell leukemia virus proteinase", BIOCHIMICA ET BIOPHYSICA ACTA. PROTEIN STRUCTURE AND MOLECULAR ENZYMOLOGY, ELSEVIER, AMSTERDAM; NL, vol. 1478, no. 1, 16 March 2000 (2000-03-16), pages 1-8, XP004278906, ISSN: 0167-4838, DOI: 10.1016/S0167-4838(99)00240-X

## Description

### RELATED APPLICATION

This application claims benefit of U.S. application Serial Number 61/809,237 filed 5 April 2013.

### TECHNICAL FIELD

Provided are methods and compositions, e.g., kits, for isolating protein or other biomolecules from biological or environmental samples containing humic substances, e.g. soil, compost, sediment, or manure samples.

### BACKGROUND

Protein sequences have a wide variety of applications in the field of molecular biology. They are a valuable tool in many analytical and application techniques used in the field of molecular biology, health and medicine, bioterrorism, forensics, space science, and food science. Some examples of these techniques include proteome mapping of microorganisms, detecting pathogens and beneficial microorganisms in soils, water, water filters, biofilms, plants and animals, and forensic identification of biological samples and environmental samples contaminated with different biological entities. All of these techniques are based on identifying a specific sequence of amino acids in either a biological sample, such as a microorganism, plant tissues or animal tissues, or in any environment capable of supporting life. Identifying target protein sequences directly in biological samples and in environmental samples has the advantages of speed, accuracy, high-throughput, and a low limit of detection of proteins. The target protein sequence, in order to be used as a diagnostic tool in such applications, should be free of contaminants that inhibit downstream applications. These contaminants are often from the groups that include phenolic and porphyrin substances, such as humic acids, fulvic acids, lignans, heme, chlorophyll, and quinones.

The field of protein extraction and subsequent analysis by mass spectrometry and other methods has enabled the rapid analysis of the composition of certain biological samples. However, the existing methods of protein extraction are often inadequate for isolation and purification of protein from environmental samples such as soil, manure, and plants that contain significant amounts of phenolic or porphyrin substances. In analysis of such samples, the protein is invariably co-extracted with the phenolic or porphyrin substances, thereby making it difficult to perform accurate analysis of the protein composition.

The nature of the contaminants in crude protein preparations from soils and sediments and their interactions with proteins are not well understood. Most frequently these contaminants are considered to be humic and fulvic acids and a heterogeneous mixture of phenolic polymers and oligomers. Humic substances are formed when microbes degrade plant residues and are stabilized to degradation by covalent binding of their reactive sites to metal ions and clay minerals. Humic substances consist of polycyclic aromatics to which saccharides, peptides, and phenols are attached. The predominant types of humic substances in soils are humic acids (molecular weight of 300 kDa and greater) and fulvic acids (molecular weight of as low as 0.1 kDa). Humic acids are soluble in alkaline pH and precipitate with hydrochloric or sulphuric acids at pH 1.0 to 2.0, while fulvic acids remain in solution even at acidic pH (Stevenson, 1994). Most frequently, protein extracts from soils showing brown coloration are indicative of contamination with humic-like substances. These brown compounds cannot be easily removed from protein extracts.

Analysis and extraction or release of biomolecules including proteins has been performed in the art (see US 2003/0153083 A1, WO 2012/135081 A2, Grasso et al. (Methods in Enzymology 2007, 430:59-78), EP 2 199 295 A1, US 5,665,359, US 7,429,648 B1, WO 2006/130720 A2, Thermo Scientific Pierce® Cell Lysis Technical Handbook (2008), Zahuczky et al. (Biochim. Biophys. Acta 2000, 1478(1): 1-8) and US 5,834,282). Different DNA extraction methods have also been described (see Yeates et al. (Biological Procedures Online 1998, 1(1):40-47), Schneegurt et al. (Curr. IssuesMol. Biol. 2003, 5:1-8), Willner et al. (PLoS ONE 2012, 7(4), e34605), WO 2009/140313 A1, WO 2009/025690 A2.

Standard methods for extraction of total protein involve thermally assisted detergent-based cellular lysis using, for example, SDS, followed by precipitation with trichloroacetic acid (TCA). An exemplary method is described by Chourey et al. (J. Proteome Res. 2010, 9(12):6615-22). Direct extraction of total protein from soils or sediments usually results in co-extraction of other soil components, mainly humic acids or other humic substances, which negatively interfere with protein detecting processes. Separation of humic substances from protein usually involves time-consuming and tedious steps. What is needed is a method for rapid isolation of protein from biological and environmental samples, in which the protein is effectively separated from the humic substances in the sample.

### SUMMARY

The invention is defined in the appended claims 1 to 24.

In one aspect, the present invention provides a method for purifying or isolating protein from a sample comprising cells and one or more humic substance contaminants, wherein the sample is a soil sample, the method comprising the steps of the steps of:
(a) contacting the soil sample with a first solution comprising at least 0.1 vol% non-ionic detergent, at least 10mM Tris buffer, 10 to 100 mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts; and
(b) contacting the resulting mixture of step (a) with a second solution comprising 1 to 6M inorganic salt, wherein the inorganic salt in the second solution is selected from sodium salts, potassium salts, calcium salts, magnesium salts, chloride salts, bicarbonate salts and sulfate salts, and wherein the total salt concentration in the mixture after the addition of the second solution is from 0.2 to 1M; and
(c) recovering protein from the mixture.

In another aspect, the present invention provides a method for removing one or more humic substance contaminants from a soil sample, the method comprising the steps of:
(a) contacting the soil sample with a first solution comprising at least 0.1 vol% non-ionic detergent, at least 10mM Tris buffer, 10 to 100 mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts; and
(b) contacting the resulting mixture of step (a) with a second solution comprising 1 to 6M inorganic salt, wherein the inorganic salt in the second solution is selected from sodium salts, potassium salts, calcium salts, magnesium salts, chloride salts, bicarbonate salts and sulfate salts, and wherein the total salt concentration in the mixture after the addition of the second solution is from 0.2 to 1M; and
(c) removing the one or more humic substance contaminants.

In a further aspect, the present invention provides a method for purifying protein from a sample comprising cells and one or more humic substance contaminants, wherein the sample is a soil sample, the method comprising the steps of:
(a) contacting the sample with a first solution comprising at least 10mM Tris Base, EDTA, 10 to 100mM KCl, 10 to 100 mM MgCl₂, at least 1 vol% glycerol, and at least 0.1 vol% Triton X-100;
(b) contacting the resulting mixture of step (a) with a second solution comprising 1 to 6M sodium chloride, wherein the total salt concentration in the mixture after the addition of the second solution is from 0.2 to 1M; and
(c) removing the one or more humic substance contaminants.

In another aspect, the present invention provides a kit for performing a method according to any one of claims 1-20, comprising
(a) a first solution comprising at least 0.1 vol% non-ionic detergent, at least 10mM Tris buffer, 10 to 100mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts;
(b) a second solution comprising 1 to 6M inorganic salt, wherein the inorganic salt in the second solution is selected from sodium salts, potassium salts, calcium salts, magnesium salts, chloride salts, bicarbonate salts and sulfate salts; and
(c) homogenizing beads, optionally wherein the beads are glass beads, ceramic beads or a mixture thereof.

In a further aspect the present invention pertains to the use of a kit for performing a method according to any one of claims 1-20, the kit comprising
(a) a first solution comprising at least 0.1 vol% non-ionic detergent, at least 10mM Tris buffer, 10 to 100mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts; and
(b) a second solution comprising 1 to 6M inorganic salt, wherein the inorganic salt in the second solution is selected from sodium salts, potassium salts, calcium salts, magnesium salts, chloride salts, bicarbonate salts and sulfate salts.

Also disclosed is a method for removing one or more contaminants from a biological or environmental sample, the method comprising the steps of: (a) contacting the sample with a first solution comprising a detergent, a buffer, one or more inorganic salts, and a polyol; and (b) contacting the resulting mixture of step (a) with a second solution comprising an inorganic salt. In some embodiments, the second solution comprises an inorganic salt which is sodium chloride. In some embodiments, the first solution further comprises a chelating agent. In some embodiments, the first solution further comprises an anti-foaming agent. In some embodiments, the first solution comprises two or more inorganic salts. In some embodiments, the method further comprises contacting the sample with a disulfide-reducing agent following step (a). In some variations the disulfide-reducing agent is DTT (dithiothreitol). In some embodiments, the method further comprises the step of agitating the resulting mixture of step (a), for example, in the presence of beads. In some embodiments, the method further comprises the step of agitating the resulting mixture of step (b), for example, in the presence of beads.

Also disclosed is a method for removing one or more contaminants from a biological or environmental sample, the method comprising the steps of: (a) contacting the sample with a first solution comprising a detergent, a buffer, one or more inorganic salts, and a polyol; (b) agitating the resulting mixture of step (a) in the presence of beads; (c) contacting the resulting mixture of step (b) with a second solution comprising an inorganic salt; and (d) agitating the resulting mixture of step (c) in the presence of beads. In some embodiments, the first solution further comprises a chelating agent. In some embodiments, the first solution further comprises an anti-foaming agent. In some embodiments, the second solution comprises an inorganic salt which is sodium chloride. In some embodiments, the method further comprises contacting the sample with a disulfide-reducing agent following step (a). In some variations, the disulfide-reducing agent is DTT.

Also disclosed is a method for purifying or isolating a biomolecule, e.g., protein, DNA, RNA, or lipid, from a sample comprising cells and one or more contaminants, the method comprising extracting the biomolecule into a solution, wherein the biomolecule is at least partially soluble in the solution, and the one or more contaminants are at least partially insoluble in the solution. In some embodiments, the method further comprises the step of lysing the cells. In some embodiments, the method further comprises the step of agitating the sample. In some embodiments, the method further comprises contacting the sample with a disulfide-reducing agent (e.g., DTT). In some embodiments, the biomolecule is partially soluble in the solution. In some embodiments, the biomolecule is mostly soluble in the solution. In some embodiments, the biomolecule is fully soluble in the solution. In some embodiments, the one or more contaminants are partially insoluble in the solution. In some embodiments, the one or more contaminants are mostly insoluble in the solution. In some embodiments, the one or more contaminants are fully insoluble in the solution.

Also disclosed is a method for purifying or isolating a biomolecule, e.g., protein, DNA, RNA, or lipid, from a sample comprising cells and one or more contaminants, the method comprising the steps of: (a) contacting the sample with a first solution comprising a detergent, a buffer, one or more inorganic salts, and a polyol; and (b) contacting the resulting mixture of step (a) with a second solution comprising an inorganic salt. In some embodiments, the first solution further comprises a chelating agent. In some embodiments, the first solution further comprises an anti-foaming agent. In some embodiments, the first solution comprises two or more inorganic salts. In some embodiments, the method further comprises contacting the sample with a disulfide-reducing agent (e.g., DTT) following step (a). In some embodiments, the method further comprises the step of agitating the resulting mixture of step (a) in the presence of beads. In some embodiments, the method further comprises the step of agitating the resulting mixture of step (b) in the presence of beads.

Also disclosed is a method for purifying or isolating a biomolecule, e.g., protein, DNA, RNA, or lipid, from a sample comprising cells and one or more contaminants, the method comprising the steps of: (a) contacting the sample with a first solution comprising a detergent, a buffer, one or more inorganic salts, and a polyol; (b) agitating the resulting mixture of step (a) in the presence of beads; and (c) contacting the resulting mixture of step (b) with a second solution comprising an inorganic salt; and (d) agitating the resulting mixture of step (c) in the presence of beads. In some embodiments, the first solution further comprises a chelating agent. In some embodiments, the first solution further comprises an anti-foaming agent. In some embodiments, the second solution comprises an inorganic salt which is sodium chloride. In some embodiments, the method further comprises contacting the sample with a disulfide-reducing agent (e.g., DTT) following step (a).

Also disclosed is a method for purifying or isolating a biomolecule, e.g., protein DNA, RNA, or lipid, from a sample comprising cells and one or more contaminants, the method comprising the steps of: (a) contacting the sample with a first solution comprising Tris Base, EDTA, KCl, MgCl₂, glycerol, and Triton X-100; and (b) contacting the resulting mixture of step (a) with a second solution comprising sodium chloride. In some embodiments, the first solution further comprises an anti-foaming agent.

Also disclosed is a method for purifying or isolating a biomolecule, e.g., protein DNA, RNA, or lipid, from a sample comprising cells and one or more contaminants, the method comprising the steps of: (a) contacting the sample with a first solution comprising Tris Base, EDTA, KCl, MgCl₂, glycerol, and Triton X-100; (b) agitating the resulting mixture of step (a) in the presence of beads; (c) contacting the resulting mixture of step (b) with a second solution comprising sodium chloride; and (d) agitating the resulting mixture of step (c) in the presence of beads. In some embodiments, the beads are glass or ceramic beads. In some embodiments, the first solution further comprises an anti-foaming agent.

Also disclosed is a kit comprising (a) a first solution comprising a detergent, a buffer, one or more inorganic salts, and a polyol; and (b) a second solution comprising an inorganic salt. In some embodiments, the first solution further comprises a chelating agent. In some embodiments, the first solution further comprises an anti-foaming agent. In some embodiments, the second solution comprises an inorganic salt which is sodium chloride. In some embodiments, the kit further comprises instructions describing a method for use according to any of the methods described herein. In some embodiments, the kit further comprises beads. In some embodiments, the kit further comprises an apparatus that can be used to agitate a sample in the presence of beads. In some embodiments, the kit further comprises an adaptor for connecting a vessel containing the sample to a vortex apparatus (e.g., Vortex Adapter, Mo Bio Laboratories, Carlsbad, CA). In one aspect, the kit comprises one or more tube vessels useful for performing the method of use. Where tube vessels are included in the kit, the vessels can be sterile. In some embodiments, the kit includes components useful for further processing an isolated biomolecule, e.g., protein, DNA, RNA, or lipid.

Also disclosed is a kit comprising (a) a first solution comprising Tris Base, EDTA, KCl, MgCl₂, glycerol, and Triton X-100, (b) a second solution comprising sodium chloride, and (c) glass or ceramic beads. In some embodiments, the kit further comprises instructions describing a method of use according to any of the methods described herein. In some embodiments, the first solution further comprises an anti-foaming agent.

### BRIEF DESCRIPTION OF THE DRAWINGS

Figure 1 shows an SDS-PAGE gel of *E. coli*-spiked sterile soils prepared according to the procedure described in Example 1.
Figure 2 shows protein extracted from 5 g of *E. coli*-spiked soil using thermally assisted detergent-based cellular lysis with SDS, followed by TCA precipitation.

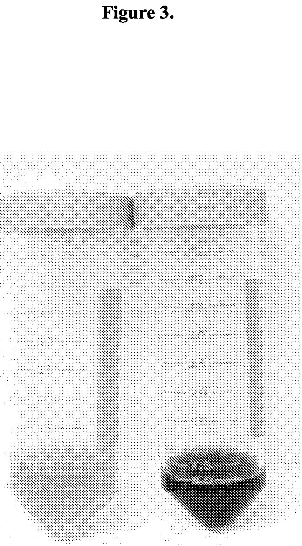

Figure 3 shows crude protein extracts prior to protein precipitation. The extract on the left was prepared using the procedure described in Example 1. The extract on the right was prepared using the procedure described by Chourey et al. (*supra*)*.* The darker color of the extract on the right is a result of co-extraction of humic substances. Proteins were extracted from 5 g of an organically rich soil.

### DETAILED DESCRIPTION

Provided are methods and compositions for detecting and/or isolating a biomolecule, e.g., protein, DNA, RNA, or lipid, and/or for detecting an organism, e.g., a microorganism, in a sample, e.g., a biological or environmental sample. Provided are methods and compositions, e.g., kits, for isolating a biomolecule from sources containing contaminating substances that interfere with use of the purified biomolecules in subsequent applications. In one aspect, provided are methods and kits for purifying a biomolecule, e.g., protein, DNA, RNA, or lipid, from a biological or environmental sample to be free of contaminants that may impede analysis or identification of the biomolecules. The environmental samples include but are not limited to soil, sediment, sludge, decomposing biological matter, archaeological remains, peat bogs, compost and water that are terrestrial or subterranean in origin. Biomolecules isolated using the kits and methods provided herein may be used in the areas of molecular biological application, including, for example, analytical, cloning, diagnostic, and detection in the fields of agriculture, horticulture, forestry, forensics, biological research, organism and sample composition identification, characterization, applied microbiology, proteomics, environmental analysis, water testing, and combating bioterrorism.

The present invention is defined in the appended claims 1 to 24.

As used herein, unless clearly indicated otherwise, the terms "a," "an," "the," and the like refer to one or more.

As used herein, the terms "including," "containing," and "comprising" are used in their open, non-limiting sense.

To provide a more concise description, some of the quantitative expressions given herein are not qualified with the term "about." It is understood that, whether the term "about" is used explicitly or not, every quantity given herein is meant to refer to that actual given value, and it is also meant to refer to the approximation to such given value that would reasonably be inferred based on the ordinary skill in the art, including equivalents and approximations due to the experimental and/or measurement conditions for such given value.

The term "biological sample" as used herein, refers to a sample obtained from a biological subject, including sample of biological tissue or fluid origin obtained *in vivo* or *in vitro.* Such samples can be, but are not limited to, body fluid (e.g., blood, blood plasma, serum, or urine), organs, tissues, stool, swab samples, fractions and cells isolated from mammals (e.g., humans), biofilms (e.g., oral biofilms, environmental biofilms), filtered water samples. Biological samples also may include sections of the biological sample including tissues (e.g., sectional portions of an organ or tissue). The term "biological sample" may also include extracts from a biological sample, for example, an antigen from a biological fluid (e.g., blood or urine). A biological sample may be of prokaryotic origin (e.g., bacteria, archaea) or eukaryotic origin (e.g., fungi, plants, insects, protozoa, birds, fish, reptiles). In some embodiments, the biological sample is mammalian (e.g., rat, mouse, cow, dog, donkey, guinea pig, or rabbit). In certain embodiments, the biological sample is of primate origin (e.g., example, chimpanzee or human).

The terms "environmental" and "environmental sample", include any environmental material, e.g., material contained in the earth and space, including space dust, airborne and waterborne locations and will include any organism, structure, and component considered alive, dead, dormant or inactive, whole, complete, undecaying and decaying that contains a biomolecule, e.g., protein, DNA, RNA, or lipid. "Environmental" and "environmental sample" include material and organisms that may be isolated from the environment as dust or suspended material collected by filtration.

The term "soil" as used herein refers to environmental samples of soil, sediment, manure, compost, and the like, e.g., commercial potting mixtures, commercial soil amendments. The term also includes a broad range of organic carbon and nitrogen content and varying sand, silt and/or clay compositions. "Soil" includes any composition containing components commonly associated with habitable and uninhabitable areas of the earth and space, including for example varying descriptions, e.g., indoor dust, outdoor dust, dirt, mud, muck, silt, ground, sewage, compost, composting landfills at various depths. Examples of soil samples include but are not limited to landfill (e.g., 0-3 inches deep or 3-6 inches deep); late-stage compost; coffee compost; marine sediment; lake sediment; mud sediment; animal manure (e.g., horse manure); mulch, e.g., mulch top soil; the ocean floor, hillsides, mountaintops and may extend from the surface to any depth. The sample may be collected by any means using any commercially available or improvised method and tested directly. A biomolecule, e.g., protein, DNA, RNA, or lipid, may be extracted using a kit or method provided herein at the site of collection, or the sample may be stored before a biomolecule, e.g., protein, DNA, RNA, or lipid, is isolated therefrom.

### Methods and Compositions

In some aspects of any of the methods and compositions described herein, the one or more contaminants include, without limitation, humic substances, such as humic acids, fulvic acids, and lignans, heme, chlorophyll, and quinones. In some embodiments, contaminants include phenolic or porphyrin compounds other than proteins, oligopeptides, amino acids, DNA, RNA, oligonucleotides, nucleic acids, and lipids. In some embodiments, contaminants are phenolic or porphyrin components of natural organic matter in soil and water as well as in geological organic deposits such as lake sediments, peats, brown coals, and shales. In some embodiments, contaminants are complex and heterogeneous mixtures of polydispersed materials formed by reactions during the decay and transformation of plant and microbial remains and may be derived from components such as plant lignin, polysaccharides, melanin, cutin, proteins, lipids, nucleic acids, and fine char particles.

In some aspects of any of the methods and compositions described herein, the first solution is added to the sample prior to addition of the second solution. The first solution may be added to the sample at room temperature, or it may be added at a temperature below room temperature. The sample may be kept on ice or otherwise kept below room temperature before, during, or after addition of the first solution. In some instances, a disulfide-reducing agent (e.g., dithiothreitol) is added to the solution following addition of the first solution and prior to addition of the second solution. Addition of the first solution, optionally followed by addition of a disulfide-reducing agent, may create a hypotonic environment for the sample.

In some variations, the sample is agitated by any of the methods described herein after addition of the first solution or after addition of the disulfide-reducing agent. Agitation may be for 1, 2, 5, 10, 15, 20, 25, 30, 40, 50, or 60 minutes. Agitation may be carried out at room temperature or at a temperature below room temperature, for example, at 4 °C. Agitation at low temperature may be desirable for native protein extraction. In some variations, the sample may be incubated on at room temperature or at a temperature below room temperature, for example, at 4 °C, prior to or following agitation. Incubation may be for 1, 2, 5, 10, 15, 20, 25, 30, 40, 50, or 60 minutes.

In some variations, the sample is centrifuged prior to addition of the second solution. Centrifugation may help remove residual soil, beads, buffer, or other components of the sample solution from the sides or cap of the vessel containing the solution before adding the second solution. The sample may be centrifuged at room temperature or at a temperature below room temperature, for example, at 4 °C. The sample may be centrifuged in a refrigerated centrifuge.

In some variations, the sample is agitated by any of the methods described herein after addition of the second solution or after addition of the disulfide-reducing agent. Agitation may be for 1, 2, 5, 10, 15, 20, 25, 30, 40, 50, or 60 minutes. Agitation may be carried out at room temperature or at a temperature below room temperature, for example, at 4 °C. Agitation at low temperature may be desirable for native protein extraction. In some variations, the sample may be incubated on at room temperature or at a temperature below room temperature, for example, at 4 °C, prior to or following agitation. Incubation may be for 1, 2, 5, 10, 15, 20, 25, 30, 40, 50, or 60 minutes.

In some variations, the sample is centrifuged following addition of the second solution. Centrifugation may help separate the extracted biomolecule, e.g., protein, DNA, RNA, or lipid, from the soil particles, bead, and other undissolved material in the vessel. The sample may be centrifuged at room temperature or at a temperature below room temperature, for example, at 4 °C. The sample may be centrifuged in a refrigerated centrifuge. Following centrifugation, the supernatant may be collected and centrifuged an additional time. This may help to remove fine soil particles and other undissolved material from the extracted biomolecule, e.g., protein, DNA, RNA, or lipid.

In some aspects of any of the methods and compositions described herein, the first solution comprises a detergent, a buffer, one or more inorganic salts, and a polyol. In some variations, the first solution further comprises a chelating agent. In some variations, the first solution further comprises an anti-foaming agent.

In some embodiments, the first solution contains a detergent. The detergent may be any non-ionic detergent. Particular detergents that can be used in the compositions and methods described herein include, without limitation, Triton X-100, Triton X-114, Brij 58, Brij 35, sodium taurocholate, Tween 20, Tween 80, polysorbate 20, polysorbate 80, NP-40 (nonyl phenoxypolyethoxylethanol), CHAPS 3-[(3-cholamidopropyl)dimethylammonio]-1-propanesulfonate), and CHAPSO (3-[(3-cholamidopropyl)dimethylammonio]-2-hydroxy-1-propanesulfonate). The first solution may contain 0.01 to 2, 0.1 to 1, 0.5 to 1, 0.5 to 1.5 or 0.75 to 1.25 vol% detergent. The first solution may contain at least about 0.01, about 0.1, about 0.5, about 0.75, or about 1 vol% detergent. The first solution may contain up to about 0.1, about 0.25, about 0.5, about 0.75, about 1, about 1.5, or about 2 vol% detergent. In some instances, the first solution contains 0.1 to 1 vol% Triton X-100 detergent. In other instances, the first solution contains about 1 vol% Triton X-100 detergent.

In some embodiments, the first solution contains a chelating agent. The chelating agent may be EDTA (ethylenediaminetetraacetic acid), or it may be a salt form of EDTA, such as a sodium, potassium, or calcium salt of EDTA. The first solution may contain 0.01 to 4, 0.1 to 3, 0.1 to 2, 1 to 2, 1.5 to 2.5, 0.1 to 1, 0.5 to 2, or 0.5 to 1 mM chelating agent. The first solution may contain at least about 0.01, about 0.1, about 0.25, about 0.5, about 0.75, about 1, about 1.25, about 1.5, about 2, or about 3 mM chelating agent. The first solution may contain up to about 0.1, about 0.25, about 0.5, about 0.75, about 1, about 1.25, about 1.5, about 2, about 3, or about 4 mM chelating agent. In some instances, the first solution contains 0.1 to 2 mM EDTA. In other instances, the first solution contains about 2 mM EDTA.

In some embodiments of the subject matter described herein, the first solution contains a buffer. The buffer may be a basic buffer. The buffer may be any buffer that is amenable to culturing cells. The buffer may be any buffer that is useful for maintaining the first solution at physiological pH. Particular buffers that can be used in the compositions and methods described herein include, without limitation, Tris Base (tris(hydroxymethyl)aminomethane), Bis-Tris (Bis(2-hydroxyethyl)-amino-tris(hydroxymethyl)-methane), HEPES (4-(2-hydroxyethyl)-1-piperazineethanesulfonic acid), PBS (phosphate buffered saline), MOPS (3-(N-morpholino)propanesulfonic acid), MES (2-(N-morpholino)ethanesulfonic acid), and CAPS (N-cyclohexyl-3-aminopropanesulfonic acid). The first solution may contain 1 to 200, 10 to 100, 10 to 80, 1 to 50, 1 to 20, 10 to 50, 10 to 30, 10 to 20, 20 to 50, or 15 to 25 mM buffer. The first solution may contain at least about 1, about 10, about 20, about 30, about 40, about 50, about 60, about 70, about 80, about 90, or about 100 mM buffer. The first solution may contain up to about 10, about 20, about 30, about 40, about 50, about 60, about 70, about 80, about 90, about 100, about 150, or about 200 mM buffer. In some instances, the first solution contains 10 to 100 mM Tris Base buffer. In other instances, the first solution contains about 20 mM Tris Base buffer.

In some embodiments of the subject matter described herein, the first solution contains a polyol. In some variations, the polyol is a disaccharide. Particular polyols that can be used in the compositions and methods described herein include, without limitation, glycerol, sucrose, and trehalose. In a particular variation, the polyol is glycerol. The first solution may contain 1 to 30, 5 to 20, 5 to 10, 10 to 20, 5 to 15, or 10 to 15 vol% polyol. The first solution may contain at least about 1, about 5, about 10, about 15, about 20, or about 25 vol% polyol. The first solution may contain up to about 5, about 10, about 15, about 20, about 25, or about 30 vol% polyol. In some instances, the first solution contains 5 to 20 vol% glycerol. In other instances, the first solution contains 10 vol% glycerol.

In some embodiment of the subject matter described herein, the first solution contains one or more salts. The salts may be inorganic salts, such as sodium salts, potassium salts, calcium salts, magnesium salts, chloride salts, bicarbonate salts, or sulfate salts. In some variations, the salts contain monovalent cations or divalent cations. Particular salts that can be used in the compositions and methods described herein include, without limitation, KCl, NaCl, NaHCO₃, NaSO₄, MgCl₂, and CaCl₂. In some embodiments, the first solution contains two or more different salts, such as a potassium salt and a magnesium salt. In some embodiments, the first solution contains one salt selected from MgCl₂ and CaCl₂, and one salt selected from KCl, NaCl, NaHCO₃, and NaSO₄. In some instances, the first solution contains MgCl₂ and KCl. In some variations, the first solution contains CaCl₂ and does not contain NaCl. The first solution may contain 1 to 300, 10 to 250, 20 to 200, 10 to 100, 20 to 50, 10 to 30, or 20 to 40 mM salt. The first solution may contain at least about 1, about 10, about 20, about 30, about 40, about 50, about 60, about 70, about 80, about 90, about 100, about 150, about 200, or about 250 mM salt. The first solution may contain up to about 10, about 20, about 30, about 40, about 50, about 60, about 70, about 80, about 90, about 100, about 150, about 200, about 250, or about 300 mM salt. In some instances, the first solution contains 10 to 100 mM salt selected from KCl, NaCl, NaHC03, and NaSO₄, and 10 to 100 mM salt selected from MgCl₂ and CaCl₂. In some instances, the first solution contains 10 to 100 mM KCl and 10 to 100 mM MgCl₂. In other instances, the first solution contains about 10 mM KCl and about 10 mM MgCl₂.

In some embodiments of the subject matter described herein, the first solution contains an anti-foaming agent. The anti-foaming agent may be a silica-based anti-foaming agent. Particular anti-foaming agents that can be used in the compositions and methods described herein include 100% active silicone, poly(methylsiloxane) in silicone oil, or silicone emulsions. Particular silicone emulsions contain from 10 to 30% silicone and one or more emulsifiers.

In some variations, the first solution contains Tris Base, KCl, MgCl₂, glycerol, Triton X-100, and EDTA. In a particular variation, the first solution contains about 20 mM Tris Base, about 2 mM EDTA, about 10 mM KCl, about 10 mM MgCl₂, about 10% glycerol, and about 1% Triton X-100.

In some variations of the subject matter described herein, the second solution contains one or more salts. The salts may be inorganic salts, such as sodium salts, potassium salts, calcium salts, magnesium salts, chloride salts, bicarbonate salts, or sulfate salts. In some variations, the salts contain monovalent cations or divalent cations. In some variations, the salts contain monovalent cations. Particular salts that can be used in the compositions and methods described herein include, without limitation, KCl, NaCl, NaHCO₃, NaSO₄, MgCl₂, and CaCl₂. In some embodiments, the first solution contains a sodium salt or a potassium salt. In some embodiments, the first solution contains a chloride salt. In some embodiments, the second solution contains NaCl. In some embodiments, the second solution contains MgCl₂. In some embodiment, the second solution contains CaCl₂. The second solution may contain 1 to 6, 2 to 5, 3 to 5, 4 to 5, 3.5 to 4.5, or 4 to 4.5 M salt. The second solution may contain at least about 1, about 2, about 3, about 4, or about 5 M salt. The second solution may contain up to about 1, about 2, about 3, about 4, about 5 or about 6 M salt. In some variations, the total concentration of salt in the sample after addition of the second solution is 0.01 to 1, 0.1 to 0.5, 0.2 to 0.4, 0.1 to 0.3, or 0.3 to 0.5 M. In some variations, the total concentration of salt in the sample after addition of the second solution is at least about 0.01, about 0.1, about 0.2, about 0.3, about 0.4, about 0.5, about 0.6, or about 0.7 M. In some variations, the total concentration of salt in the sample after addition of the second solution is up to about 0.1, about 0.2, about 0.3, about 0.4, about 0.5, about 0.6, about 0.7 M, or about 1 M. In some variations, the second solution contains 4.2 M NaCl. In some variations, the total concentration of NaCl in the sample after addition of the second solution is about 140 to 500 mM.

The methods and compositions described herein may comprise one or more steps or components for agitation of the sample. Agitation may be achieved by any method known in the art, including, without limitation, sonication, blending, mechanical homogenization (e.g., shear homogenization, rotor-stator homogenization), manual homogenization (e.g., mortar and pestle or dounce homogenization), or high pressure homogenization (e.g., French Pressure Cell). Agitation may be carried out at room temperature or at a temperature below room temperature. In some embodiments, the sample is agitated at about 4 °C. In some embodiments, the sample is agitated in the presence of beads (i.e. "bead beating"). The beads may be homogenizing beads. The beads may be made of any solid material that is nonreactive with the samples, solutions, or other reagents used in the method. The beads may be round or irregularly shaped. The beads may be of uniform size or of varying sizes. The beads may be of uniform material or of heterogeneous material. In some variations, the beads are ceramic. In some variations, the beads are glass. In some variations, the beads have an average diameter of 0.01 to 10, 0.1 to 5, 0.1 to 3, 0.2 to 3, 0.1 to 2, or 1 to 3 mm. In some variation the beads have an average diameter of at least about 0.01, about 0.1, about 0.2, about 0.5, about 1, about 2, about 3, or about 5 mm. In some variations, the beads have an average diameter of up to about 0.1, about 0.2, about 0.5, about 1, about 2, about 3, about 5, or about 10 mm. In some instances, the beads are 0.2, 1.4, or 2.8 mm ceramic beads. In some instances, the beads are 0.1 or 0.5 mm glass beads. In a particular variation, the beads are 0.2 mm ceramic beads. In another particular variation, the beads are 0.1 mm glass beads. In yet another particular variation, the beads are a mixture of 0.1 mm glass beads and 0.2 mm ceramic beads. Agitation of the sample in the presence of beads may be achieved by physical force, such as shaking or vibration. Vibration can be introduced by any convenient means, such as by a sonication or a vortex apparatus using a Vortex Adapter (Mo Bio Laboratories, Carlsbad, CA), for example.

The methods and compositions described herein can be used to remove one or more contaminants from a sample that contains a biomolecule, e.g., protein, DNA, RNA, or lipid. The resulting a biomolecule, e.g., protein, DNA, RNA, or lipid, may contain less than 40%, less than 30%, less than 20%, less than 10%, less than 5%, less than 2%, less than 1%, less than 0.1%, less than 0.01%, less than 0.001%, or less than 0.0001% by weight of contaminants. In some instances, the purity of the resulting protein or other biomolecule is at least 50%, at least 60%, at least 70%, at least 80%, at least 90%, at least 95%, at least 98%, at least 99%, at least 99.9%, or at least 99.99% by weight. In some instances, at least 50%, at least 60%, at least 70%, at least 80%, at least 90%, at least 95%, at least 98%, at least 99%, at least 99.9%, or at least 99.99% by weight of the contaminants present in the sample are removed using any of the methods or compositions described herein.

Any of the methods and compositions described herein can be applied to a biological or environmental sample of any scale. In some variations, the sample is a 10, 50, 100, or 500 mg sample. In some variations, the sample is a 1, 2, 5, 10, 20, 50, 100, 500, or 1,000 gram sample.

The methods and compositions described herein may be used to recover a biological molecule from a biological or environmental sample. The biological molecule may be a protein, DNA, RNA, or lipid. In some variations, the protein recovered from the sample is denatured protein. Addition of DTT to the sample (e.g., to a final concentration of 10 mM) following addition of the first solution may facilitate recovery of denatured protein. Agitation of the sample in the presence of beads following addition of the first solution to the sample and/or following addition of the second solution to the sample may also facilitate recovery of denatured protein. In other variations, the protein recovered from the sample is native protein or extracellular protein. Addition of DTT to the sample (e.g., to a final concentration of 1 mM) and/or addition of protease inhibitors following addition of the first solution may facilitate recovery of native or extracellular protein. For recovery of native or extracellular protein, agitation of the sample in the presence of beads may be for a shortened time period, or the agitation step may be absent.

Any of the methods described herein may also include subsequent steps to further separate, isolate, or purify a biomolecule, e.g., protein, DNA, RNA, or lipid, from the contaminants. For example, the solution containing a dissolved biomolecule, e.g., protein, DNA, RNA, or lipid, can be substantially removed from contact with the undissolved portions of the sample and any other components with which it is in contact, such as homogenizing beads, via methods known in the art. Such methods include filtration (e.g., microfiltration, ultrafiltration) and centrifugation (e.g., ultracentrifugation). In some instances, multiple centrifugation and/or filtration steps may be used. Centrifugation or filtration may be carried out at a temperature below room temperature (e.g., 4 °C) to minimize degradation of the biomolecule, e.g., protein, DNA, RNA, or lipid.

After filtration or centrifugation, the biomolecule, e.g., protein, DNA, RNA, or lipid, may be precipitated out of the solution or supernatant by addition of a precipitation agent, such as trichloroacetic acid (TCA). For complete precipitation to occur, incubation for 10 minutes to 24 hours may be required. Subsequent washing with acetone or other organic solvent may be performed to remove residual TCA and/or detergent. The sample may be pelleted using centrifugation. The washing and pelleting steps may be repeated multiple times.

### Applications

The methods and compositions described herein have many medical and veterinary applications, e.g., for diagnosis, prognosis, epidemiology, inspection of contamination of materials (e.g., drugs, dressing, instruments, implants), foods (e.g., inspections of meat, vegetables, seafood, etc.), including medical and veterinary analysis of feces (including manure analysis for animals). Medical and veterinary applications include detection of soils, e.g., for bioterrorism purposes, e.g., anthrax, viruses, nematodes, and the like. Virus detection using the compositions and methods provided herein can also be used to analyze manure and soil, water, water filters, biofilms, air and the like. Viruses that can be detected by compositions and methods provided herein include enterovirus, norovirus, variola, varicella, reovirus, retroviruses (e.g., HIV), viral hemorrhagic fevers (e.g., Ebola, Marburg, Machupo, Lassa), *Variola major*, viral encephalitis and the like, as listed in Table 1, below. The compositions and methods provided herein can also be used to detect spores, toxins and biologically produced poisons, for example, by detecting *Bacillus anthracis*, anthrax spores are also detected (albeit, indirectly), detection of *Clostridium perferinges* implies presence of toxin, etc.

Examples of Gram negative bacteria that can be detected and/or whose biomolecules, e.g. proteins, DNA, RNA, or lipids, can be isolated using the kits and methods provided herein include but are not limited to Gram negative rods (e.g., anaerobes such as bacteroidaceae (e.g., *Bacteroides fragilis*), facultative anaerobes, enterobacteriaceae (e.g., *Escherichia coli*), vibrionaceae (e.g., *Vibrio cholerae*), pasteurellae (e.g., *Haemophilus influenzae*), and aerobes such as pseudomonadaceae (e.g., *Pseudomonas aeruginosa*); Gram negative cocci (e.g., aerobes such as Neisseriaceae (e.g., *Neisseria meningitidis*) and Gram negative obligate intracellular parasites (e.g., Rickettsiae (e.g., *Rickettsia* spp.). Examples of Gram negative bacteria families that can be detected and/or whose biomolecules, e.g. proteins, DNA, RNA, or lipids, can be isolated include but are not limited to Acetobacteriaceae, Alcaligenaceae, Bacteroidaceae, Chromatiaceae, Enterobacteriaceae, Legionellaceae, Neisseriaceae, Nitrobacteriaceae, Pseudomonadaceae, Rhizobiaceae, Rickettsiaceae and Spirochaetaceae.

Examples of Gram positive bacteria that can be detected and/or whose biomolecules, e.g. proteins, DNA, RNA, or lipids, can be isolated using the kits and methods provided herein include but are not limited to *A. globiformis, B. subtilis, C. renale, M. luteus, R. erythropolis,* Ea39, Ben-28 and *S. lividans.* Gram positive bacteria that can be detected and/or whose biomolecules, e.g. proteins, DNA, RNA, or lipids, can be isolated also are in groups that include, for example, Corynebacterium, Mycobacterium, Nocardia; Peptococcus (e.g., *P. niger*); Peptostreptococcus (e.g., *Ps. anaerobius*; some species in the group form clumps and clusters; some species in the group form diplococci (the latter of which are distinguished by their ability to form butyrate); and some species in the group are capable of fermentation, reduction of nitrate, production of indole, urease, coagulase or catalase); Ruminococcus; Sarcina; Coprococcus; Arthrobacter (e.g., *A. globiformis, A. citreus* or *A. nicotianae*); Micrococcus (e.g., *M. luteus* (previously known as *M. lysodeikticus*), *M. lylae, M. roseus*, *M. agilis*, *M. kristinae* and *M. halobius*); Bacillus (e.g., *B. anthracis, B. azotoformans*, *B. cereus*, *B. coagulans*, *B. israelensis*, *B. larvae*, *B. mycoides, B. polymyxa*, *B. pumilis, B. stearothormophillus*, *B. subtilis*, *B. thuringiensis*, *B. validus, B. weihenstephanensis* and *B. pseudomycoides*); Sporolactobacillus; Sporocarcina; Filibacter; Caryophanum and Desulfotomaculum. Other Gram positive bacteria that can be detected and/or whose biomolecules, e.g. proteins, DNA, RNA, or lipids, can be isolated fall into the group *Clostridium,* which often include peritrichous flagellation, often degrade organic materials to acids, alcohols, CO₂, H₂ and minerals (acids, particularly butyric acid, are frequent products of clostridial fermentation), and in one aspect form ellipsoidal or spherical endospores, which may or may not swell the sporangium. Species of *Clostridium* that can be detected and/or whose nucleic acid can be isolated include psychrophilic, mesophilic or thermophilic species, saccharolytic species, proteolytic species and/or specialist species, and those that are both saccharolytic and proteolytic species. Saccharolytic species of *Clostridium* that can be detected and/or whose biomolecules, e.g. proteins, DNA, RNA, or lipids, can be isolated include but are not limited to *Cl. aerotolerans*, *Cl. aurantibutyricum*, *Cl. beijerinckii*, *Cl. botulinum B*,*E*,*F**, *Cl. butyricum*, *Cl. chauvoei*, *Cl. difficile*, *Cl. intestinale*, *Cl. novyi A, Cl. pateurianum*, *Cl. saccharolyticum*, *Cl. septicum*, *Cl. thermoaceticum*, and *Cl. thermosaccharolyticum.*

Proteolytic species of *Clostridium* that can be detected and/or whose biomolecules, e.g. proteins, DNA, RNA, or lipids, can be isolated include but are not limited to *Cl. argeninense*, *Cl. ghoni*, *Cl. limosum*, *Cl. putrefaciens*, *Cl. subterminale* and *Cl. tetani.* Species that are proteolytic and saccharolytic that can be detected and/or whose biomolecules, e.g. proteins, DNA, RNA, or lipids, can be isolated include but are not limited to *Cl. acetobutylicum*, *Cl. bifermenans*, *Cl. botulinum A*, *B, F (prot.)**, *Cl. botulinum C*,*D**, *Cl. cadaveris*, *Cl. haemolyticum*, *Cl. novyi B*, *C*, **Cl. perfringens*, *Cl. putrefaciens*, *Cl. sordelli* and *Cl. sporogenes.* As indicated by an asterisk, *Cl. botulinum* is subdivided into a number of types according to the serological specificities of the toxins produced. Specialist *Clostridium* species that can be detected and/or whose biomolecules, e.g. proteins, DNA, RNA, or lipids, can be isolated include but are not limited to *Cl. acidiurici*, *Cl. irregularis*, *Cl. kluyveri*, *Cl. oxalicum*, *Cl. propionicum*, *Cl. sticklandii* and *Cl. villosum.* These specificities are based on neutralization studies. Other Clostridium species that can be detected and/or whose biomolecules, e.g. proteins, DNA, RNA, or lipids, can be isolated include those that produce botulinum toxins.

Examples of fungi that can be detected and/or whose biomolecules, e.g. proteins, DNA, RNA, or lipids, can be isolated using the kits and methods provided herein include but are not limited to *Halocyphina villosa*, *Hypoxylon oceanicum*, *Verruculina enalia*, *Nia vibrissa*, *Antennospora quadricornuta*, *Lulworthia* spp. and *Aigialus parvus.* Examples of algae that can be detected and/or whose biomolecules, e.g. proteins, DNA, RNA, or lipids, can be isolated include but are not limited to brown algae (e.g., Phylum Phaeophycota *Dictyota* sp. (Class Phaeophyceae, Family Dictyotaceae); green algae (e.g., Phylum Chlorophycota *Chaetomorpha gracilis* (Class Chlorophyceae, Family Cladophoraceae); and red algae (e.g., Phylum Rhodophycota, *Catenella* sp. (Class Rhodophyceae, Family Rhabdoniaceae).

Organisms that can be detected by the kits and methods provided herein in a sample, e.g., an agricultural soil, include but are not limited to *Pseudomonas* spp., *Serratia* spp., *Bacillus* spp., *Flavobacterium* spp., Actinomycetes and fungi; in polluted soils include but are not limited to *Pseudomonas* spp. and *Xanthomonas* spp.; in marsh/sediments include but are not limited to *Escherichia* spp., *Proteus* spp., Methanogens and Actinomycetes; and in forest soils include but are not limited to Mycorrhizae, Fungi and Actinomycetes. An example of a bacterium detected in soil samples for use in combating bioterrorism using methods and kits provided herein is *Bacillus anthracis.*

Pathogens and toxins that can be detected by kits and methods provided herein include, without limitation, those listed in Table 1, below:

**Table 1**

| **Disease** / **Type** | **Organism / agent** | **CDC Group** | **Specific Type** | **General Class** | **Detection Type** | **1° Target** |
|---|---|---|---|---|---|---|
| Anthrax | Bacillus anthracis | A | G+ Spore | Bacterium | DNA | Human |
| Plague | Yersinia pestis | A | G-Veg | Bacterium | DNA | Human |
| Tularemia | Francisella tularensis | A | G- Veg | Bacterium | DNA | Human |
| Brucellosis | Brucella spp. | B | G-Veg | Bacterium | DNA | Human |
| Glanders | Burkholderia mallei | B | G- Veg | Bacterium | DNA | Human |
| Melioidosis | Burkholderia pseudomallei | B | G-Veg | Bacterium | DNA | Human |
| Psittacosis | Chlamydia psittaci | B | G- Veg | Bacterium | DNA | Human |
| Q Fever | Coxiella burnettii | B | Gv Veg | Bacterium | DNA | Human |
| Typhus fever | Rickettsia prowazekii | B | Gv Veg | Bacterium | DNA | Human |
| Smallpox | Variola major | A | Virus | Virus | DNA | Human |
| Viral hemorrhagic fevers | Ebola | A | Filovirus | Virus | RNA | Human |
| Viral hemorrhagic fevers | Marburg | A | Filovirus | Virus | RNA | Human |
| Viral hemorrhagic fevers | Machupo | A | Arenavirus | Virus | RNA | Human |
| Viral hemorrhagic fevers | Lassa | A | Arenavirus | Virus | RNA | Human |
| Viral encephalitis | Venezuelan Equine Encephalitis | B | Alphavirus | Virus | RNA | Human |
| Viral encephalitis | Eastern Equine Encephalitis | B | Alphavirus | Virus | RNA | Human |
| Viral encephalitis | Western Equine Encephalitis | B | Alphavirus | Virus | RNA | Human |
| Viral infection | Echovirus | N/A | Enterovirus | Virus | RNA/ Protein | Human |
| Poliomyelitis | Poliovirus | N/A | Enterovirus | Virus | RNA/ Protein | Human |
| Common cold | Rhinovirus | N/A | Enterovirus | Virus | RNA/ Protein | Human |
| Hand, food, and mouth disease | Coxsackie A virus | N/A | Enterovirus | Virus | RNA/ Protein | Human |
| Viral infection | Coxsackie B virus | N/A | Enterovirus | Virus | RNA/ Protein | Human |
| Viral infection | Other Enteroviruses | N/A | Enterovirus | Virus | RNA/ Protein | Human |
| Viral gastroenteritis | Norovirus | N/A | Calicivirus | Virus | RNA/ Protein | Human |
| Hepatitis A | Hepatitis A virus | N/A | Picornavirus | Virus | RNA/ Protein | Human |
| Hepatitis B | Hepatitis B virus | N/A | Hepadnavirus | Virus | DNA/ Protein | Human |
| Hepatitis C | Hepatitis C virus | N/A | Togavirus | Virus | RNA/ Protein | Human |
| Botulism | Clostridium botulinum toxin | A | Toxin | Toxin | Protein | Human |
| Toxins | Ricinus communis | B | Toxin | Toxin | Protein | Human |
| Toxins | Staph. aureus | B | Enterotoxin B | Toxin | Protein | Human |
| Toxins | Clostridium perferinges toxin | B | Epsilon Toxin | Toxin | Protein | Human |

The kits and methods provided herein can be used to isolate the total protein in a biological or environmental sample, or they may be used to isolate one or more specific proteins in the sample, for example, in order to assess the activity of such specific protein. The specific protein may be, for example, a fungal protein or a protein from a Gram positive bacterium. The specific protein may be an extracellular protein. It may be desirable, in some instances, to collect native (e.g., undigested) protein from the sample. In other instances, it may be desirable to collect digested protein.

Biomolecules, e.g., protein, DNA, RNA, or lipids, isolated or purified using any of the methods or compositions described herein can be detected, analyzed, characterized, and/or further purified using any method known in the art. Particular methods of detecting, analyzing, characterizing, and/or further purifying biomolecules, e.g., protein, DNA, RNA, or lipids, isolated or purified using any of the methods or compositions described herein include 1-dimensional polyacrylamide gel electrophoresis (ID PAGE), 2- dimensional polyacrylamide gel electrophoresis (2D PAGE), ELISA-type assays for assessment of native protein activity, other enzyme-based assays, western blotting, sequencing, and antibody production (e.g., injecting proteins into animals such as rabbits or making monoclonal antibodies).

The compositions and methods will be further described with reference to the following examples; however, it is to be understood that the kits and methods are not limited to such examples.

### EXAMPLES

### Example 1: Protein Isolation from an Environmental Sample

Sterile soil samples from various sources (lake sediment, lagoon sediment, beach sand, and agricultural soil) were spiked with *E. coli.* Five grams of a particular *E. coli*-spiked soil sample were added to 50 ml bead tubes containing a mixture of 0.1 mm glass and 0.2 mm ceramic beads and placed on ice. The first solution was added to the soil (15 ml) followed by addition of dithiothreitol (DTT) to a final concentration of 10 mM. Samples were vortexed to mix and incubated on ice for 10 minutes at 4°C. Following ice incubation, sample tubes were vortexed for 10 minutes with the vortex set to the highest speed. A MO BIO Vortex Adapter for 50 ml tubes was utilized for the bead beating step. Samples were collected in the tube by brief centrifugation in a refrigerated centrifuge at 4°C. The second solution was added (1.5 ml) and samples vortexed to mix and incubated on ice at 4°C for 30 minutes. This was followed by a second round of bead beating on the vortex in the 50 ml tube adapters for 10 minutes at the highest setting. Samples were collected by centrifugation at 4500 x g, in a refrigerated centrifuge set at 4°C for 20 minutes.

The supernatant containing the protein was transferred to a clean 50 ml centrifuge tube and centrifuged again at 4500 x g in refrigerated centrifuge set at 4°C for 20 minutes. The supernatant was transferred to a clean 50 ml centrifuge tube. Samples were precipitated using 0.25 ml of 100% trichloroacetic acid (TCA) to each 1 ml of supernatant, vortexed to mix, and incubated overnight at -20°C.

The precipitated protein was recovered by centrifugation of the 50 ml tubes at 4500 x g for 20 minutes. The supernatant was discarded. The protein pellets were washed by resuspension in 1 ml of ice cold HPLC-grade acetone and pelleted by centrifugation at 4500 x g for 10 minutes. The acetone was decanted and the wash step was repeated using 1 ml of ice cold acetone. Protein was pelleted by centrifugation at 4500 x g for 10 minutes. The wash step was repeated a third time and the final pellets were dried in a biological safety hood or with N₂ gas.

The final protein pellets were resuspended in 200 µl of Tris-HCl, pH 8.45. 10µL of the suspension were combined with 10 µL of Laemmli buffer, heated at 70 °C for 10 minutes, and then loaded onto an SDS-PAGE gel.

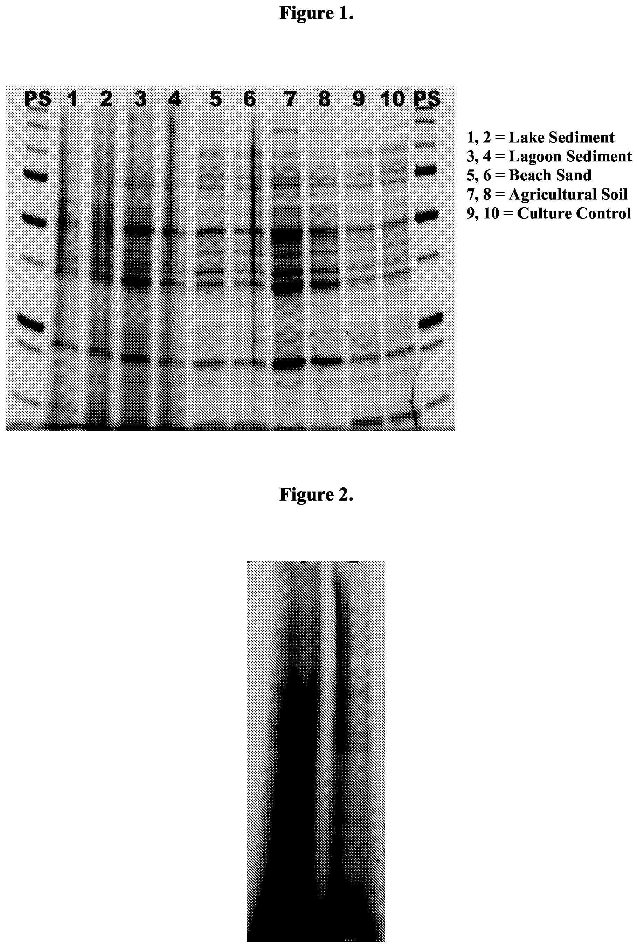

Figure 1 shows the SDS-PAGE gel of the final protein collected from each sample, as well as an *E. coli* culture control sample. For comparison, Figure 2 shows an SDS-PAGE gel of protein extracted from 5 g of *E. coli*-spiked soil using thermally assisted detergent-based cellular lysis with SDS, followed by TCA precipitation, as described by Chourey et al., (J. Proteome Res. 2010, 9(12): 6615-22). The gel in Figure 2 has significant background staining, which can make it difficult to see the protein bands. In contrast, the bands in the gel of Figure 1 have little to no background staining.

Modifications may be made to the foregoing without departing from the basic aspects of the methods and compositions provided herein. Although the compositions and methods have been described in substantial detail with reference to one or more specific embodiments, those of skill in the art will recognize that changes may be made to the embodiments specifically disclosed in this application.

## Claims

1. A method for purifying or isolating protein from a sample comprising cells and one or more humic substance contaminants, wherein the sample is a soil sample, the method comprising the steps of:
(a) contacting the soil sample with a first solution comprising at least 0.1 vol% non-ionic detergent, at least 10mM Tris buffer, 10 to 100 mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts; and
(b) contacting the resulting mixture of step (a) with a second solution comprising 1 to 6M inorganic salt, wherein the inorganic salt in the second solution is selected from sodium salts, potassium salts, calcium salts, magnesium salts, chloride salts, bicarbonate salts and sulfate salts, and wherein the total salt concentration in the mixture after the addition of the second solution is from 0.2 to 1M; and
(c) recovering protein from the mixture.

2. A method for removing one or more humic substance contaminants from a soil sample, the method comprising the steps of:
(a) contacting the soil sample with a first solution comprising at least 0.1 vol% non-ionic detergent, at least 10mM Tris buffer, 10 to 100 mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts; and
(b) contacting the resulting mixture of step (a) with a second solution comprising 1 to 6M inorganic salt, wherein the inorganic salt in the second solution is selected from sodium salts, potassium salts, calcium salts, magnesium salts, chloride salts, bicarbonate salts and sulfate salts, and wherein the total salt concentration in the mixture after the addition of the second solution is from 0.2 to 1M; and
(c) removing the one or more humic substance contaminants.

3. The method according to claim 2, comprising obtaining a solution containing dissolved protein and recovering protein therefrom.

4. The method of any one of claims 1 - 3, wherein the first solution comprises a chelating agent.

5. The method of any one of claims 1 -4, wherein the first solution comprises an anti-foaming agent.

6. The method of any of claims 1-5, wherein the second solution comprises an inorganic salt which is sodium chloride.

7. The method of any one of claims 1-6, further comprising contacting the sample with a disulfide-reducing agent following step (a).

8. The method of any one of claims 1-7, further comprising one or both of:
(i) agitating the resulting mixture of step (a), preferably in the presence of beads; and
(ii) agitating the resulting mixture of step (b), preferably in the presence of beads.

9. The method according to any one of claims 1-8, wherein the sample is agitated after addition of the first solution or after addition of the disulfide-reducing agent, optionally wherein
(i) agitation is carried out at room temperature or at a temperature below room temperature, such as 4°C; and/or
(ii) wherein the sample is incubated at room temperature or at a temperature below room temperature, such as 4°C, prior to or following agitation.

10. The method according to any one of claims 1 to 9, wherein the sample is agitated after addition of the second solution or after addition of the disulfide-reducing agent, optionally wherein
(i) agitation is carried out at room temperature or at a temperature below room temperature, such as 4°C; and/or
(ii) wherein the sample is incubated at room temperature or at a temperature below room temperature, such as 4°C, prior to or following agitation.

11. The method according to any one of claims 1 to 10, further comprising one or both of:
(i) centrifuging the resulting mixture of step (a); and
(ii) centrifuging the resulting mixture of step (b).

12. The method according to any one of claims 1 - 11, wherein the method comprises centrifuging the sample after addition of the second solution to separate undissolved material from the extracted protein.

13. The method according to any one of claims 1- 12, comprising obtaining a solution that comprises the dissolved protein and wherein the protein is recovered from the solution by adding a precipitation agent to precipitate the protein out of the solution, optionally wherein the solution comprising the dissolved protein is obtained by removing undissolved material by filtration or centrifugation.

14. The method according to any one of claims 1 - 13, wherein the first solution comprises:
(i) 0.1 to 2% by volume of non-ionic detergent;
(ii) 10 to 200 mM Tris buffer;
(iii) 0.01 to 4 mM of a chelating agent;
(iv) 10 to 100 mM of one or more inorganic salts; and
(v) 1 to 30 % by volume of a polyol.

15. The method according to any one of claims 1 -14, wherein step (a) comprises contacting the sample with a first solution comprising Tris Base, EDTA, KCl, MgCl₂, glycerol, and Triton X-100; and wherein the second solution used in step (b) comprises sodium chloride.

16. The method of any one of claims 1 -15, comprising one or both of:
(i) agitating the resulting mixture of step (a) in the presence of ceramic beads, glass beads or a mixture thereof; and
(ii) agitating the resulting mixture of step (b) in the presence of ceramic beads, glass beads or a mixture thereof.

17. A method for purifying protein from a sample comprising cells and one or more humic substance contaminants, wherein the sample is a soil sample, the method comprising the steps of:
(a) contacting the sample with a first solution comprising at least 10mM Tris Base, EDTA, 10 to 100mM KCl, 10 to 100 mM MgCl₂, at least 1 vol% glycerol, and at least 0.1 vol% Triton X-100;
(b) contacting the resulting mixture of step (a) with a second solution comprising 1 to 6M sodium chloride, wherein the total salt concentration in the mixture after the addition of the second solution is from 0.2 to 1M; and
(c) removing the one or more humic substance contaminants.

18. The method according to any one of claims 1 -17, wherein the first solution comprises 10 to 100 mM Tris Base, 0.1 to 2 mM EDTA, 10 to 100 mM KCl, 10 to 100 mM MgCl₂, 5 to 20 % by volume glycerol, and 0.1 to 1 % by volume Triton X-100, optionally wherein said first solution contains 20mM Tris base, 2mM EDTA, 10mM KCl, 10mM MgCl₂, 10% by volume glycerol and 1% Triton X-100.

19. The method according to any one of claims 1 -18, wherein the second solution comprises 2 to 5 M or 4 to 4.5 M NaCl.

20. The method according to any one of claims 1 -19, wherein the soil sample is selected from sediment, manure, compost, dust, dirt, mud, ground, landfill and mulch and/or the one or more humic substances are selected from humic acids and fulvic acids.

21. A kit for performing a method according to any one of claims 1-20, comprising
(a) a first solution comprising at least 0.1 vol% non-ionic detergent, at least 10mM Tris buffer, 10 to 100mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts;
(b) a second solution comprising 1 to 6M inorganic salt, wherein the inorganic salt in the second solution is selected from sodium salts, potassium salts, calcium salts, magnesium salts, chloride salts, bicarbonate salts and sulfate salts; and
(c) homogenizing beads, optionally wherein the beads are glass beads, ceramic beads or a mixture thereof.

22. The kit according to claim 21, wherein the first solution has one or more of the following characteristics:
(i) the first solution comprises:
(aa) 0.1 to 2% by volume of non-ionic detergent;
(bb) 10 to 200 mM Tris buffer;
(cc) 0.01 to 4 mM of a chelating agent;
(dd) 10 to 100 mM of one or more inorganic salts; and
(ee) 1 to 30 % by volume of a polyol;
(ii) the first solution comprises Tris Base, EDTA, KCl, MgCl₂, glycerol, and Triton X-100, optionally wherein the first solution comprises 10 to 100 mM Tris Base, 0.1 to 2 mM EDTA, 10 to 100 mM KCl, 10 to 100 mM MgCl₂, 5 to 20 % by volume glycerol, and 0.1 to 1% by volume Triton X-100;
and/or
(iii) the first solution comprises an anti-foaming agent.

23. The kit according to claim 21 or 22, having one or more of the following characteristics:
(i) the second solution comprising sodium chloride, optionally wherein the second solution comprises 2 to 5 M NaCl;
(ii) the beads are glass beads, ceramic beads or a mixture thereof;
(iii) the kit comprises a sterile tube vessel.

24. Use of a kit for performing a method according to any one of claims 1-20, the kit comprising
(a) a first solution comprising at least 0.1 vol% non-ionic detergent, at least 10mM Tris buffer, 10 to 100mM salt wherein the first solution comprises one or more inorganic salts, and at least 1 vol% polyol, wherein the one or more inorganic salts in the first solution is selected from sodium salts, potassium salts, calcium salts, magnesium salts, chloride salts, bicarbonate salts and sulfate salts; and
(b) a second solution comprising 1 to 6M inorganic salt, wherein the inorganic salt in the second solution is selected from sodium salts, potassium salts, calcium salts, magnesium salts, chloride salts, bicarbonate salts and sulfate salts.

## Patentansprüche

1. Verfahren zum Aufreinigen oder Isolieren von Protein aus einer Probe, die Zellen und eine oder mehrere verunreinigende Huminstoffe umfasst, wobei die Probe eine Bodenprobe ist, das Verfahren umfassend die Schritte:
(a) In Kontakt bringen der Bodenprobe mit einer ersten Lösung, umfassend mindestens 0,1 Vol.-% nicht-ionisches Detergens, mindestens 10 mM Tris-Puffer, 10 bis 100 mM Salz, wobei die erste Lösung ein oder mehrere anorganische Salze umfasst, und mindestens 1 Vol.-% Polyol, wobei das eine oder die mehreren anorganischen Salze in der ersten Lösung ausgewählt sind aus Natriumsalzen, Kaliumsalzen, Kalziumsalzen, Magnesiumsalzen, Chloridsalzen, Bicarbonatsalzen und Sulfatsalzen; und
(b) In Kontakt bringen der aus Schritt (a) resultierenden Mischung mit einer zweiten Lösung, umfassend 1 bis 6 M anorganisches Salz, wobei das anorganische Salz in der zweiten Lösung ausgewählt ist aus Natriumsalzen, Kaliumsalzen, Kalziumsalzen, Magnesiumsalzen, Chloridsalzen, Bicarbonatsalzen und Sulfatsalzen, und wobei die Gesamt-Salzkonzentration in der Mischung nach Zugabe der zweiten Lösung von 0,2 bis 1 M ist; und
(c) Erhalten von Protein aus der Mischung.

2. Verfahren zum Entfernen eines oder mehrerer verunreinigender Huminstoffe aus einer Bodenprobe, das Verfahren umfassend die Schritte:
(a) In Kontakt bringen der Bodenprobe mit einer ersten Lösung umfassend mindestens 0,1 Vol.-% nicht-ionisches Detergens, mindestens 10 mM Tris-Puffer, 10 bis 100 mM Salz, wobei die erste Lösung ein oder mehrere anorganische Salze umfasst, und mindestens 1 Vol.-% Polyol, wobei das eine oder die mehreren anorganischen Salze in der ersten Lösung ausgewählt sind aus Natriumsalzen, Kaliumsalzen, Kalziumsalzen, Magnesiumsalzen, Chloridsalzen, Bicarbonatsalzen und Sulfatsalzen; und
(b) In Kontakt bringen der aus Schritt (a) resultierenden Mischung mit einer zweiten Lösung, umfassend 1 bis 6 M anorganisches Salz, wobei das anorganische Salz in der zweiten Lösung ausgewählt ist aus Natriumsalzen, Kaliumsalzen, Kalziumsalzen, Magnesiumsalzen, Chloridsalzen, Bicarbonatsalzen und Sulfatsalzen, und wobei die Gesamt-Salzkonzentration in der Mischung nach Zugabe der zweiten Lösung von 0,2 bis 1 M ist; und
(c) Entfernen des einen oder der mehreren verunreinigenden Huminstoffe.

3. Das Verfahren nach Anspruch 2, umfassend Erhalten einer Lösung, die gelöstes Protein enthält, und Erhalten von Protein daraus.

4. Das Verfahren nach einem der Ansprüche 1 - 3, wobei die erste Lösung einen Komplexbildner umfasst.

5. Das Verfahren nach einem der Ansprüche 1 - 4, wobei die erste Lösung ein Antischaummittel umfasst.

6. Das Verfahren nach einem der Ansprüche 1 - 5, wobei die zweite Lösung ein anorganisches Salz, das Natriumchlorid ist, umfasst.

7. Das Verfahren nach einem der Ansprüche 1 - 6, weiter umfassend in Kontakt bringen der Probe mit einem Disulfid-Reduktionsmittel nach Schritt (a).

8. Das Verfahren nach einem der Ansprüche 1 - 7, weiter umfassend eines oder beide von:
(i) Bewegen der aus Schritt (a) resultierenden Mischung, vorzugsweise in der Gegenwart von Beads; und
(ii) Bewegen der aus Schritt (b) resultierenden Mischung, vorzugsweise in der Gegenwart von Beads.

9. Das Verfahren nach einem der Ansprüche 1 - 8, wobei die Probe nach Zugabe der ersten Lösung oder nach Zugabe des Disulfid-Reduktionsmittels bewegt wird, wobei gegebenenfalls
(i) bei Raumtemperatur oder bei einer Temperatur unter Raumtemperatur, wie beispielsweise 4°C, bewegt wird; und/oder
(ii) die Probe bei Raumtemperatur oder bei einer Temperatur unter Raumtemperatur, wie beispielsweise 4°C, vor oder nach dem Bewegen inkubiert wird.

10. Das Verfahren nach einem der Ansprüche 1 - 9, wobei die Probe nach Zugabe der zweiten Lösung oder nach Zugabe des Disulfid-Reduktionsmittels bewegt wird, wobei gegebenenfalls
(i) bei Raumtemperatur oder bei einer Temperatur unter Raumtemperatur, wie beispielsweise 4°C, bewegt wird; und/oder
(ii) die Probe bei Raumtemperatur oder bei einer Temperatur unter Raumtemperatur, wie beispielsweise 4°C, vor oder nach dem Bewegen inkubiert wird.

11. Das Verfahren nach einem der Ansprüche 1 - 10, weiter umfassend eines oder beide von:
(i) Zentrifugieren der aus Schritt (a) resultierenden Mischung; und
(ii) Zentrifugieren der aus Schritt (b) resultierenden Mischung.

12. Das Verfahren nach einem der Ansprüche 1 - 11, wobei das Verfahren Zentrifugieren der Probe nach Zugabe der zweiten Lösung umfasst, um ungelöstes Material von dem extrahierten Protein zu trennen.

13. Das Verfahren nach einem der Ansprüche 1 - 12, umfassend Erhalten einer Lösung, die das gelöste Protein umfasst, und wobei das Protein aus der Lösung durch Zugabe eines Fällungsmittels, um das Protein aus der Lösung auszufällen, erhalten wird, wobei gegebenenfalls die das gelöste Protein umfassende Lösung durch Entfernen von ungelöstem Material durch Filtration oder Zentrifugation erhalten wird.

14. Das Verfahren nach einem der Ansprüche 1 - 13, wobei die erste Lösung umfasst:
(i) 0,1 bis 2 Vol.-% nicht-ionisches Detergens;
(ii) 10 bis 200 mM Tris-Puffer;
(iii) 0,01 bis 4 mM eines Komplexbildners;
(iv) 10 bis 100 mM eines oder mehrerer anorganischer Salze; und
(v) 1 bis 30 Vol.-% eines Polyols.

15. Das Verfahren nach einem der Ansprüche 1 - 14, wobei Schritt (a) das in Kontakt bringen der Probe mit einer ersten Lösung, die Tris-Base, EDTA, KCl, MgCl₂, Glycerol und Triton X-100 enthält, umfasst; und wobei die zweite in Schritt (b) verwendete Lösung Natriumchlorid enthält.

16. Das Verfahren nach einem der Ansprüche 1 - 15, umfassend eines oder beide von:
(i) Bewegen der aus Schritt (a) resultierenden Mischung in der Gegenwart von Keramik-Beads, Glas-Beads oder einer Mischung davon; und
(ii) Bewegen der aus Schritt (b) resultierenden Mischung in der Gegenwart von Keramik-Beads, Glas-Beads oder einer Mischung davon.

17. Verfahren zum Aufreinigen von Protein aus einer Probe, die Zellen und eine oder mehrere verunreinigende Huminstoffe umfasst, wobei die Probe eine Bodenprobe ist, das Verfahren umfassend die Schritte:
(a) in Kontakt bringen der Probe mit einer ersten Lösung, die mindestens 10 mM Tris-Base, EDTA, 10 bis 100 mM KCl, 10 bis 100 mM MgCl₂, mindestens 1 Vol.-% Glycerol, und mindestens 0,1 Vol.-% Triton X-100 umfasst;
(b) in Kontakt bringen der aus Schritt (a) resultierenden Mischung mit einer zweiten Lösung, umfassend 1 bis 6 M Natriumchlorid, wobei die Gesamt-Salzkonzentration in der Mischung nach Zugabe der zweiten Lösung von 0,2 bis 1 M ist; und
(c) Entfernen des einen oder der mehreren verunreinigenden Huminstoffe.

18. Das Verfahren nach einem der Ansprüche 1 - 17, wobei die erste Lösung 10 bis 100 mM Tris-Base, 0,1 bis 2 mM EDTA, 10 bis 100 mM KCl, 10 bis 100 mM MgCl₂, 5 bis 20 Vol.-% Glycerol und 0,1 bis 1 Vol.-% Triton X-100 umfasst, wobei die erster Lösung gegebenenfalls 20 mM Tris-Base, 2 mM EDTA, 10 mM KCl, 10 mM MgCl₂, 10 Vol.-% Glycerol und 1% Triton X-100 enthält.

19. Das Verfahren nach einem der Ansprüche 1 - 18, wobei die zweite Lösung 2 bis 5 M oder 4 bis 4,5 M NaCl umfasst.

20. Das Verfahren nach einem der Ansprüche 1 - 19, wobei die Bodenprobe ausgewählt ist aus Sediment, Dung, Kompost, Staub, Schmutz, Schlamm, Erde, Deponieauffüllung und Mulch, und/oder die einen oder mehreren Huminstoffe ausgewählt sind aus Huminsäuren und Fulvinsäuren.

21. Kit zum Durchführen eines Verfahrens nach einem der Ansprüche 1 - 20, umfassend
(a) eine erste Lösung umfassend mindestens 0,1 Vol.-% nicht-ionisches Detergens, mindestens 10 mM Tris-Puffer, 10 bis 100 mM Salz, wobei die erste Lösung ein oder mehrere anorganische Salze umfasst, und mindestens 1 Vol.-% Polyol, wobei das eine oder die mehreren anorganischen Salze in der ersten Lösung ausgewählt sind aus Natriumsalzen, Kaliumsalzen, Kalziumsalzen, Magnesiumsalzen, Chloridsalzen, Bicarbonatsalzen und Sulfatsalzen;
(b) eine zweite Lösung, umfassend 1 bis 6 M anorganisches Salz, wobei das anorganische Salz in der zweiten Lösung ausgewählt ist aus Natriumsalzen, Kaliumsalzen, Kalziumsalzen, Magnesiumsalzen, Chloridsalzen, Bicarbonatsalzen und Sulfatsalzen; und
(c) Homogenisierungs-Beads, wobei die Beads gegebenenfalls Glas-Beads, Keramik-Beads oder eine Mischung davon sind.

22. Das Kit nach Anspruch 21, wobei die erste Lösung eine oder mehrere der folgenden Eigenschaften aufweist:
(i) die erste Lösung umfasst:
(aa) 0,1 bis 2 Vol.-% nicht-ionisches Detergens;
(bb) 10 bis 200 mM Tris-Puffer;
(cc) 0,01 bis 4 mM eines Komplexbildners;
(dd) 10 bis 100 mM eines oder mehrerer anorganischer Salze; und
(ee) 1 bis 30 Vol.-% eines Polyols;
(ii) die erste Lösung umfasst Tris-Base, EDTA, KCl, MgCl₂, Glycerol unnd Triton X-100, wobei die erste Lösung gegebenenfalls 10 bis 100 mM Tris-Base, 0,1 to 2 mM EDTA, 10 bis 100 mM KCl, 10 bis 100 mM MgCl₂, 5 bis 20 Vol.-% Glycerol und 0,1 bis 1 Vol.-% Triton X-100 umfasst;
und/oder
(iii) die erste Lösung umfasst ein Antischaummittel.

23. Das Kit nach Anspruch 21 oder 22, aufweisend eine oder mehrere der folgenden Eigenschaften:
(i) die zweite Lösung umfasst Natriumchlorid, wobei die zweite Lösung gegebenenfalls 2 bis 5 M NaCl umfasst;
(ii) die Beads sind Glas-Beads, Keramik-Beads oder eine Mischung davon;
(iii) das Kit umfasst ein steriles Rohrgefäß.

24. Verwendung eines Kits zum Durchführen eines Verfahrens nach einem der Ansprüche 1 - 20, das Kit umfassend
(a) eine erste Lösung umfassend mindestens 0,1 Vol.-% nicht-ionisches Detergens, mindestens 10 mM Tris-Puffer, 10 bis 100 mM Salz, wobei die erste Lösung ein oder mehrere anorganische Salze umfasst, und mindestens 1 Vol.-% Polyol, wobei das eine oder die mehreren anorganischen Salze in der ersten Lösung ausgewählt sind aus Natriumsalzen, Kaliumsalzen, Kalziumsalzen, Magnesiumsalzen, Chloridsalzen, Bicarbonatsalzen und Sulfatsalzen; und
(b) eine zweite Lösung, umfassend 1 bis 6 M anorganisches Salz, wobei das anorganische Salz in der zweiten Lösung ausgewählt ist aus Natriumsalzen, Kaliumsalzen, Kalziumsalzen, Magnesiumsalzen, Chloridsalzen, Bicarbonatsalzen und Sulfatsalzen.

## Revendications

1. Procédé destiné à purifier ou à isoler une protéine à partir d'un échantillon comprenant des cellules et un ou plusieurs contaminants de substances humiques, où l'échantillon est un échantillon de sol, le procédé comprenant les étapes consistant à :
(a) mettre en contact l'échantillon de sol avec une première solution comprenant au moins 0,1 % en volume de détergent non ionique, au moins 10 mM de tampon Tris, de 10 à 100 mM de sel où la première solution comprend un ou plusieurs sels inorganiques, et au moins 1 % en volume de polyol, où les un ou plusieurs sels inorganiques dans la première solution sont sélectionnés parmi des sels de sodium, des sels de potassium, des sels de calcium, des sels de magnésium, des sels de chlorure, des sels de bicarbonate et des sels de sulfate ; et
(b) mettre en contact le mélange résultant de l'étape (a) avec une deuxième solution comprenant de 1 à 6 M de sel inorganique, où le sel inorganique dans la deuxième solution est sélectionné parmi des sels de sodium, des sels de potassium, des sels de calcium, des sels de magnésium, des sels de chlorure, des sels de bicarbonate et des sels de sulfate, et où la concentration totale en sels dans le mélange après l'ajout de la deuxième solution va de 0,2 à 1 M ; et
(c) extraire une protéine du mélange.

2. Procédé destiné à éliminer un ou plusieurs contaminants de substances humiques d'un échantillon de sol, le procédé comprenant les étapes consistant à :
(a) mettre en contact l'échantillon de sol avec une première solution comprenant au moins 0,1 % en volume de détergent non ionique, au moins 10 mM de tampon Tris, de 10 à 100 mM de sel où la première solution comprend un ou plusieurs sels inorganiques, et au moins 1 % en volume de polyol, où les un ou plusieurs sels inorganiques dans la première solution sont sélectionnés parmi des sels de sodium, des sels de potassium, des sels de calcium, des sels de magnésium, des sels de chlorure, des sels de bicarbonate et des sels de sulfate ; et
(b) mettre en contact le mélange résultant de l'étape (a) avec une deuxième solution comprenant de 1 à 6 M de sel inorganique, où le sel inorganique dans la deuxième solution est sélectionné parmi des sels de sodium, des sels de potassium, des sels de calcium, des sels de magnésium, des sels de chlorure, des sels de bicarbonate et des sels de sulfate, et où la concentration totale en sels dans le mélange après l'ajout de la deuxième solution va de 0,2 à 1 M ; et
(c) éliminer les un ou plusieurs contaminants de substances humiques.

3. Procédé selon la revendication 2, comprenant le fait d'obtenir une solution contenant une protéine dissoute et d'en extraire une protéine.

4. Procédé selon l'une quelconque des revendications 1 à 3, dans lequel la première solution comprend un agent de chélation.

5. Procédé selon l'une quelconque des revendications 1 à 4, dans lequel la première solution comprend un agent anti-moussant.

6. Procédé selon l'une quelconque des revendications 1 à 5, dans lequel la deuxième solution comprend un sel inorganique, lequel est le chlorure de sodium.

7. Procédé selon l'une quelconque des revendications 1 à 6, comprenant en outre le fait de mettre en contact l'échantillon avec un agent réducteur de disulfure après l'étape (a).

8. Procédé selon l'une quelconque des revendications 1 à 7, comprenant en outre l'un ou les deux des faits suivants :
(i) le fait d'agiter le mélange résultant de l'étape (a), de préférence en présence de billes ; et
(ii) le fait d'agiter le mélange résultant de l'étape (b), de préférence en présence de billes.

9. Procédé selon l'une quelconque des revendications 1 à 8, dans lequel l'échantillon est agité après l'ajout de la première solution ou après l'ajout de l'agent réducteur de disulfure, optionnellement où
(i) l'agitation est effectuée à température ambiante ou à une température inférieure à la température ambiante, par exemple à 4 °C ; et/ou
(ii) où l'échantillon est incubé à température ambiante ou à une température inférieure à la température ambiante, par exemple à 4 °C, avant ou après l'agitation.

10. Procédé selon l'une quelconque des revendications 1 à 9, dans lequel l'échantillon est agité après l'ajout de la deuxième solution ou après l'ajout de l'agent réducteur de disulfure, optionnellement où
(i) l'agitation est effectuée à température ambiante ou à une température inférieure à la température ambiante, par exemple à 4 °C ; et/ou
(ii) où l'échantillon est incubé à température ambiante ou à une température inférieure à la température ambiante, par exemple à 4 °C, avant ou après l'agitation.

11. Procédé selon l'une quelconque des revendications 1 à 10, comprenant en outre l'un ou les deux des faits suivants :
(i) le fait de centrifuger le mélange résultant de l'étape (a) ; et
(ii) le fait de centrifuger le mélange résultant de l'étape (b).

12. Procédé selon l'une quelconque des revendications 1 à 11, où le procédé comprend le fait de centrifuger l'échantillon après l'ajout de la deuxième solution afin de séparer la matière non dissoute de la protéine extraite.

13. Procédé selon l'une quelconque des revendications 1 à 12, comprenant le fait d'obtenir une solution qui comprend la protéine dissoute et où la protéine est extraite de la solution en ajoutant un agent de précipitation afin de faire précipiter la protéine pour la séparer de la solution, optionnellement où la solution comprenant la protéine dissoute est obtenue en éliminant la matière non dissoute par filtration ou centrifugation.

14. Procédé selon l'une quelconque des revendications 1 à 13, dans lequel la première solution comprend :
(i) de 0,1 à 2 % en volume de détergent non ionique ;
(ii) de 10 à 200 mM de tampon Tris ;
(iii) de 0,01 à 4 mM d'un agent de chélation ;
(iv) de 10 à 100 mM d'un ou de plusieurs sels inorganiques ; et
(v) de 1 à 30 % en volume d'un polyol.

15. Procédé selon l'une quelconque des revendications 1 à 14, dans lequel l'étape (a) comprend le fait de mettre en contact l'échantillon avec une première solution comprenant du Tris-base, de l'EDTA, du KCl, du MgCl₂, du glycérol, et du Triton X-100 ; et où la deuxième solution utilisée lors de l'étape (b) comprend du chlorure de sodium.

16. Procédé selon l'une quelconque des revendications 1 à 15, comprenant l'un ou les deux des faits suivants :
(i) le fait d'agiter le mélange résultant de l'étape (a) en présence de billes de céramique, de billes de verre ou d'un mélange de celles-ci ; et
(ii) le fait d'agiter le mélange résultant de l'étape (b) en présence de billes de céramique, de billes de verre ou d'un mélange de celles-ci.

17. Procédé destiné à purifier une protéine à partir d'un échantillon comprenant des cellules et un ou plusieurs contaminants de substances humiques, où l'échantillon est un échantillon de sol, le procédé comprenant les étapes consistant à :
(a) mettre en contact l'échantillon avec une première solution comprenant au moins 10 mM de Tris-base, de l'EDTA, de 10 à 100 mM de KCl, de 10 à 100 mM de MgCl₂, au moins 1 % en volume de glycérol, et au moins 0,1 % en volume de Triton X-100 ;
(b) mettre en contact le mélange résultant de l'étape (a) avec une deuxième solution comprenant de 1 à 6 M de chlorure de sodium, où la concentration totale en sels dans le mélange après l'ajout de la deuxième solution va de 0,2 à 1 M ; et
(c) éliminer les un ou plusieurs contaminants de substances humiques.

18. Procédé selon l'une quelconque des revendications 1 à 17, dans lequel la première solution comprend de 10 à 100 mM de Tris-base, de 0,1 à 2 mM d'EDTA, de 10 à 100 mM de KCl, de 10 à 100 mM de MgCl₂, de 5 à 20 % en volume de glycérol, et de 0,1 à 1 % en volume de Triton X-100, optionnellement où ladite première solution contient 20 mM de Tris-base, 2 mM d'EDTA, 10 mM de KCl, 10 mM de MgCl₂, 10 % en volume de glycérol et 1 % en volume de Triton X-100.

19. Procédé selon l'une quelconque des revendications 1 à 18, dans lequel la deuxième solution comprend de 2 à 5 M ou de 4 à 4,5 M de NaCl.

20. Procédé selon l'une quelconque des revendications 1 à 19, dans lequel l'échantillon de sol est sélectionné parmi un sédiment, du fumier, du compost, de la poudre, de la poussière, de la boue, de la terre, de la terre de remblai ou de décharge et du paillis et/ou les une ou plusieurs substances humiques sont sélectionnées parmi des acides humiques et des acides fulviques.

21. Kit destiné à réaliser un procédé selon l'une quelconque des revendications 1 à 20, comprenant
(a) une première solution comprenant au moins 0,1 % en volume de détergent non ionique, au moins 10 mM de tampon Tris, de 10 à 100 mM de sel, où la première solution comprend un ou plusieurs sels inorganiques, et au moins 1 % en volume de polyol, où les un ou plusieurs sels inorganiques dans la première solution sont sélectionnés parmi des sels de sodium, des sels de potassium, des sels de calcium, des sels de magnésium, des sels de chlorure, des sels de bicarbonate et des sels de sulfate ;
(b) une deuxième solution comprenant de 1 à 6 M de sel inorganique, où le sel inorganique dans la deuxième solution est sélectionné parmi des sels de sodium, des sels de potassium, des sels de calcium, des sels de magnésium, des sels de chlorure, des sels de bicarbonate et des sels de sulfate ; et
(c) des billes d'homogénéisation, optionnellement où les billes sont des billes de verre, des billes de céramique ou un mélange de celles-ci.

22. Kit selon la revendication 21, dans lequel la première solution présente une ou plusieurs des caractéristiques suivantes :
(i) la première solution comprend :
(aa) de 0,1 à 2 % en volume de détergent non ionique ;
(bb) de 10 à 200 mM de tampon Tris ;
(cc) de 0,01 à 4 mM d'un agent de chélation ;
(dd) de 10 à 100 mM d'un ou de plusieurs sels inorganiques ; et
(ee) de 1 à 30 % en volume d'un polyol ;
(ii) la première solution comprend du Tris-base, de l'EDTA, du KCl, du MgCl₂, du glycérol, et du Triton X-100, optionnellement où la première solution comprend de 10 à 100 mM de Tris-base, de 0,1 à 2 mM d'EDTA, de 10 à 100 mM de KCl, de 10 à 100 mM de MgCl₂, de 5 à 20 % en volume de glycérol, et de 0,1 à 1 % en volume de Triton X-100 ;
et/ou
(iii) la première solution comprend un agent anti-moussant.

23. Kit selon la revendication 21 ou 22, présentant une ou plusieurs des caractéristiques suivantes :
(i) la deuxième solution comprenant du chlorure de sodium, optionnellement où la deuxième solution comprend de 2 à 5 M de NaCl ;
(ii) les billes sont des billes de verre, des billes de céramique ou un mélange de celles-ci ;
(iii) le kit comprend un récipient tubulaire stérile.

24. Utilisation d'un kit destiné à réaliser un procédé selon l'une quelconque des revendications 1 à 20, le kit comprenant
(a) une première solution comprenant au moins 0,1 % en volume de détergent non ionique, au moins 10 mM de tampon Tris, de 10 à 100 mM de sel, où la première solution comprend un ou plusieurs sels inorganiques, et au moins 1 % en volume de polyol, où les un ou plusieurs sels inorganiques dans la première solution sont sélectionnés parmi des sels de sodium, des sels de potassium, des sels de calcium, des sels de magnésium, des sels de chlorure, des sels de bicarbonate et des sels de sulfate ; et
(b) une deuxième solution comprenant de 1 à 6 M de sel inorganique, où le sel inorganique dans la deuxième solution est sélectionné parmi des sels de sodium, des sels de potassium, des sels de calcium, des sels de magnésium, des sels de chlorure, des sels de bicarbonate et des sels de sulfate.
